# EUROPEAN PATENT SPECIFICATION

(11) **EP 0 569 387 B1**
(45) Date of publication and mention of the grant of the patent: **05.04.1995**
(21) Application number: 92902650.8
(22) Date of filing: 05.12.1991
(51) Int. Cl.: C07D 471/04

(54) **RESOLUTION OF TRANS-2-(2-PYRIMIDINYL)-7-(HYDROXYMETHYL)OCTAHYDRO-2H-PYRIDO 1,2-A]PYRAZINE**
RECEMATSPALTUNG VON TRANS-2-(2-PYRIMIDINYL)-7-(HYDROXYMETHYL)OCTAHYDRO-2H-PYRIDO[1,2-A]-PYRAZIN.
DECOMPOSITION DE TRANS-2-(2-PYRIMIDINYLE)-7-(HYDROXYMETHYLE)OCTAHYDRO-2H-PYRIDO 1,2-A]PYRAZINE

(30) Priority: 31.01.1991 US 648686
(43) Date of publication of application: 18.11.1993
(73) Proprietor: PFIZER INC., New York, N.Y. 10017-5755 (US)
(72) Inventor: MURTIASHAW, Charles, W., North Stonington, CT 06359 (US)
(74) Representative: Wood, David John
(86) International application number: US9108314
(87) International publication number: WO9213858

(56) References cited:
- EP-A- 0 380 217

## Description

### Background of the Invention

The present invention relates to a process for the preparation of (7S,trans)-2-(2-pyrimidinyl)-7-(hydroxymethyl)octahydro-2H-pyrido[1,2-a]pyrazine which is used to prepare pyrazine compounds, both of which are disclosed in European Patent Application Publication Number 0380217 (A1), the pyrazine compounds being anxiolytic agents. The process involves resolution of trans-2-(2-pyrimidinyl)-7-(hydroxymethyl)octahydro-2H-pyrido[1,2-a]pyrazine using D-(-) or L-(+)tartaric acid. The present invention also relates to the tartrate salts that are formed as intermediates in the foregoing process.

### Summary of the Invention

The present invention relates to a process for separating trans-2-(2-pyrimidinyl)-7-(hydroxymethyl) octahydro-2H-pyrido[1,2-a]pyrazine which is a racemic mixture of an enantiomer of the formula 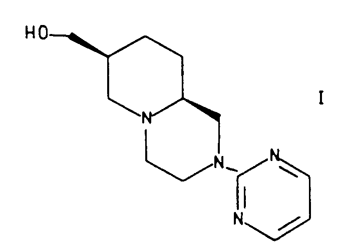
and an enantiomer of the formula 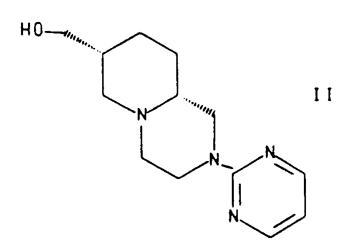
comprising reacting the racemic mixture with D (-) or L-(+)tartaric acid, separating the resulting diastereoisomer tartrate salts, and if desired, converting the tartrate salt of each enantiomer to the free base thereof.

### Detailed Description of the Invention

In the process of the present invention trans-2-(2-pyrimidinyl)-7-(hydroxymethyl)octahydro-2H-pyrido[1,2-a]pyrazine is resolved using either L-(+) or D-(-)-tartaric acid. The pyrazine is reacted with the tartaric acid in an inert polar solvent. Suitable solvents include methanol, isopropanol, ethyl acetate and tetrahydrofuran. Methanol is the preferred solvent. The temperature of the reaction is not critical. Generally, the reaction mixture will be heated to a temperature sufficient to dissolve the starting material (i.e., about 30 to about 50°C, preferably about 40°C) and then allowed to cool. Upon cooling, one of the diastereoisomer tartrate salts precipitates. When the L-(+) tartaric salt is formed the 7S enantiomer remains in solution and the 7R enantiomer precipitates. When the D-(-) tartaric salt is formed, the 7R enantiomer remains in solution and the 7S enantiomer precipitates. If the desired enantiomer remains in solution, it is recovered by evaporating the liquid.

In order to convert the salt to the free base, the salt is dissolved in water and the pH is raised to between about 10 to about 14 preferably pH 12, using base, and the base is extracted from the aqueous layer using an inert non-polar solvent, such as isopropyl ether, diethyl ether, 1,1,1-trichloroethane, or methylene chloride, preferably the latter.

For use in alleviating the symptoms of anxiety in a human subject, the pyrazine compounds disclosed in European Patent Application Publication Number 0380217 (A1) are administered in an antianxiety amount of about 2 to about 200 mg/day, in single or divided daily doses. In particular cases, dosages outside that range are prescribed at the discretion of the attending physician. The preferred route of administration is generally oral, but parenteral administration (e.g., intramuscular, intravenous, intradermal) will be preferred in special cases, e.g. where oral absorption is impaired as by disease, or the patient is unable to swallow. These compounds are generally administered in the form of pharmaceutical compositions including a pharmaceutically acceptable vehicle or diluent. Such are generally formulated in a conventional manner utilizing solid or liquid vehicles or diluents as appropriate to the mode of desired administration: for oral administration, in the form of tablets, hard or soft gelatin capsules, suspensions, granules, powders and the like; and, for parenteral administration, in the form of injectable solutions or suspensions, and the like.

The present invention is illustrated by the following examples, but is not limited to the details thereof.

### EXAMPLE 1

### (7S,trans)-2-(2-Pyrimidinyl)-7-(hydroxymethyl)octahydro-2H-pyrido[1,2-a]pyrazine

A. To a heated (60°C) solution of 50.0 g (0.201 mol) of trans-2-(2-Pyrimidinyl)-7-(hydroxymethyl)octahydro-2H-pyrido[1,2-a]pyrazine in 750 ml of methanol was added 30 g (0.201 mol) of D-(-)tartaric acid. The resulting suspension was allowed to cool to 30°C over a period of 5 hours. The solids were filtered, washed two times with 50ml of methanol and vacuum dried to provide 39.5 g (98.7%) of the desired diastereomeric salt. m.p. 201-203°C [α]_{D} = -44.2°C = 0.186, MeOH.
   The above mentioned salt (38.2 g) was slurried in refluxing methanol (395 ml) for 8 hours, cooled to 25°C and filtered. The solids were washed with 50 ml of methanol and vacuum dried to yield 34.4 g (90%) of salt. m.p. 202-204°C. [α]_{D} = -44.2°C = 0.186, MeOH.
B. The above tartrate salt (36.0 g, 0.103 mol) was dissolved in 190 ml of water, adjusted to pH 12 with 2M NaOH and extracted twice with 300 ml of methylene chloride. The combined organic phases were dried over sodium sulfate, filtered, and evaporated to provide 24.3 g (95%) of the title compound as a light brown solid, pure by NMR. [α]_{D}= -42.8° C=1.075, methanol. ¹³C NMR (300 MHz, CDCl₃): δ 161.2, 157.6, 109.7, 65.5, 60.9, 57.3, 54.8, 48.9, 43.4, 34.8, 26.1, 25.8.

### EXAMPLE 2

### (7S, trans)-2-(2-pyrimidinyl)-7-(hydroxymethyl)octahydro-2H-pyrido[1,2-a]pyrazine

A. 5.5 g (0.022 mol) of trans-2-(2-pyrimidinyl)-7-(hydroxymethyl)octahydro-2H-pyrido[1,2-a]pyrazine and 3.33 g (0.022 mol) of L-(+)tartaric acid were then added 88 ml of methanol followed by stirring at room temperature for 19 hours. After filtration, the mother liquor was evaporated under vacuum to provide 4.33 g (98%) of the (7S, trans)-2-(2-pyrimidinyl)-7-(hydroxymethyl)octahydro-2H-pyrido[1,2-a]pyrazine L-tartrate salt.
B. In order to form the free base, the tartrate salt (3.0 g, 7.54 mmol) is dissolved in 150 ml of water taken to pH 12 with 2M NaOH and extracted twice with 100 ml of methylene chloride. The combined organic phases are then dried over sodium sulfate, filtered, and evaporated to provide the free base as a light brown solid, clean by NMR. To provide an optically pure free base, the free base is then slurried in a mixture of 20 ml of methylene chloride and 20 ml of hexanes of 15 minutes, filtered, washed with hexanes and dried under vacuum to provide the title compound.

## Claims

1. A process for separating a racemic mixture of an enantiomer of the formula 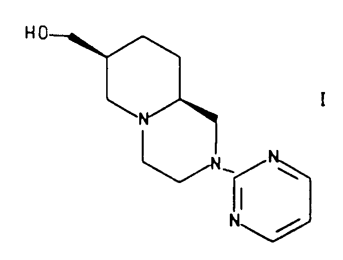 and an enantiomer of the formula 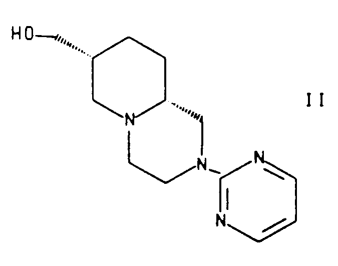 comprising:
reacting the racemic mixture with D-(-) or L-(+)tartaric acid to form the tartrate salts of each of the enantiomers;
separating each of the resulting diastereomeric tartrate salts.

2. The process of claim 1 further including, after said separating step, converting at least one of said tartrate salts to the free base thereof.

3. The process of claim 1, wherein said racemic mixture is reacted with D-(-)tartaric acid.

4. The process of claim 3, wherein said separating step includes isolating an insoluble tartrate salt from a soluble tartrate salt.

5. The process of claim 4, wherein said insoluble tartrate salt is a salt of the enantiomer of formula I.

6. The process of claim 1, wherein said racemic mixture is reacted with L-(+)tartaric acid.

7. The process of claim 6, wherein said separating step includes isolating an insoluble tartrate salt from an soluble tartrate salt.

8. The process of claim 7 wherein said insoluble tartrate salt is a salt of the enantiomer of formula II.

9. The process of claim 1 wherein said reacting step includes reading the racemic mixture with D-(-) tartaric acid to form a precipitate in a solution, said precipitate being the tartrate salt of the enantiomer of formula I;
said separating step includes separating the precipitate from the solution; and
further including converting the tartrate salt of the diastereoisomer of formula I to the free base thereof.

10. The process of claim 1 wherein said reacting step includes reacting the racemic mixture with L-(+)tartaric acid to form a precipitate in a solution, the solution including the tartrate salt of the enantiomer of formula I dissolved therein;
said separating step includes separating the precipitate from the solution and evaporating away the solution to leave the salt of the enantiomer of formula I; and
further including converting the tartrate salt of the enantiomer of formula I to the free base thereof.

11. A salt of a compound of the formula 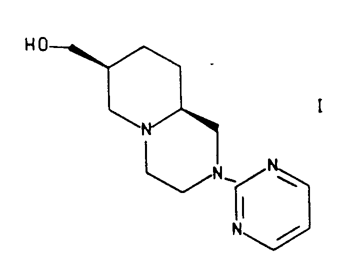 with an acid selected from the group consisting of D-(-) tartaric acid and L-(+) tartaric acid.

12. A salt of a compound of the formula 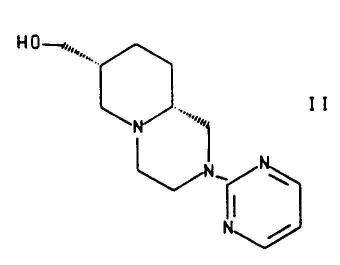 with an acid selected from the group consisting of D-(-) tartaric acid and L-(+) tartaric acid.

## Patentansprüche

1. Verfahren zur Trennung eines racemischen Gemisches aus einem Enantiomer der Formel 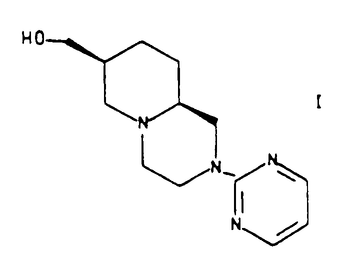 und einem Enantiomer der Formel 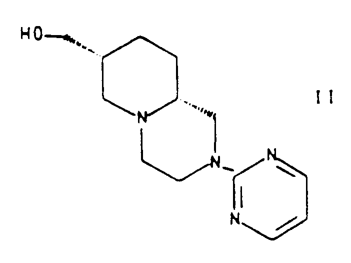 umfassend:
Umsetzen des racemischen Gemisches mit D-(-)- oder L-(+)-Weinsäure zur Bildung der Tartratsalze von jedem der Enantiomeren;
Abtrennen jedes der erhaltenen diastereomeren Tartratsalze.

2. Verfahren nach Anspruch 1, wobei des weiteren nach dem Abtrennungsschritt die Umwandlung von mindestens einem der Tartratsalze zu der freien Base davon eingeschlossen ist.

3. Verfahren nach Anspruch 1, wobei das racemische Gemisch mit D-(-)-Weinsäure umgesetzt wird.

4. Verfahren nach Anspruch 3, wobei der Abtrennungsschritt Isolieren eines unlöslichen Tartratsalzes von einem löslichen Tartratsalz einschließt.

5. Verfahren nach Anspruch 4, wobei das unlösliche Tartratsalz ein Salz des Enantiomers der Formel I ist.

6. Verfahren nach Anspruch 1, wobei das racemische Gemisch mit L-(+)-Weinsäure umgesetzt wird.

7. Verfahren nach Anspruch 6, wobei der Abtrennungsschritt Isolieren eines unlöslichen Tartratsalzes von einem löslichen Tartratsalz einschließt.

8. Verfahren nach Anspruch 7, wobei das unlösliche Tartratsalz ein Salz des Enantiomers der Formel II ist.

9. Verfahren nach Anspruch 1, wobei der Umsetzungsschritt einschließt, Umsetzen des racemischen Gemisches mit D-(-)-Weinsäure zur Bildung eines Niederschlages in einer Lösung, wobei der Niederschlag das Tartratsalz des Enantiomers der Formel I ist;
der Abtrennungsschritt Abtrennen des Niederschlags von der Lösung einschließt; und
außerdem das Umwandeln des Tartratsalzes des Diastereomers der Formel I in die freie Base davon eingeschlossen ist.

10. Verfahren nach Anspruch 1, wobei der Umsetzungsschritt einschließt, Umsetzen des Reaktionsgemisches mit L-(+)-Weinsäure zur Bildung eines Niederschlages in einer Lösung, wobei die Lösung das Tartratsalz des Enantiomers der Formel I, darin gelöst, einschließt;
der Abtrennungschritt Abtrennen des Niederschlages aus der Lösung und Verdampfen der Lösung unter Zurücklassen des Salzes des Enantiomers der Formel I einschließt; und
außerdem das Umwandeln des Tartratsalzes des Diastereomers der Formel I in die freie Base davon eingeschlossen ist.

11. Salz einer Verbindung der Formel 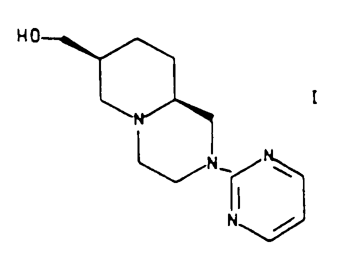 mit einer Säure, ausgewählt aus der Gruppe, bestehend aus D-(-)-Weinsäure und L-(+)-Weinsäure.

12. Salz einer Verbindung der Formel 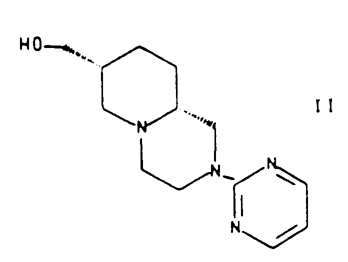 mit einer Säure, ausgewählt aus der Gruppe, bestehend aus D-(-)-Weinsäure und L-(+)-Weinsäure.

## Revendications

1. Procédé pour séparer un mélange racémique d'un énantiomère de formule 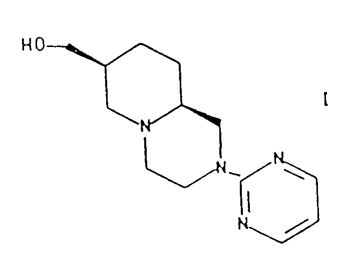 et d'un énantiomère de formule 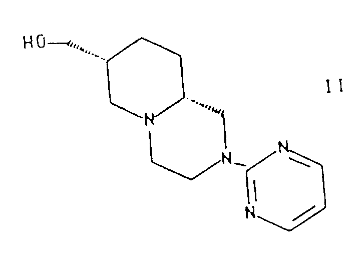 comprenant :
la réaction du mélange racémique avec l'acide D-(-)- ou L-(+)-tartrique pour former les tartrates de chacun des énantiomères ; et
la séparation de chacun des tartrates diastéréoisomères résultants.

2. Procédé suivant la revendication 1, comprenant en outre, après l'étape de séparation, la transformation d'au moins un des tartrates en la base libre correspondante.

3. Procédé suivant la revendication 1, dans lequel le mélange racémique est amené à réagir avec l'acide D-(-)-tartrique.

4. Procédé suivant la revendication 3, dans lequel l'étape de séparation comprend l'isolement d'un tartrate insoluble d'un tartrate soluble.

5. Procédé suivant la revendication 4, dans lequel le tartrate insoluble est un sel de l'énantiomère de formule I.

6. Procédé suivant la revendication 1, dans lequel le mélange racémique est amené à réagir avec l'acide L-(+)-tartrique.

7. Procédé suivant la revendication 6, dans lequel l'étape de séparation comprend l'isolement d'un tartrate insoluble d'un tartrate soluble.

8. Procédé suivant la revendication 7, dans lequel le tartrate insoluble est un sel de l'énantiomère de formule II.

9. Procédé suivant la revendication 1, dans lequel l'étape de réaction comprend la réaction du mélange racémique avec l'acide D-(-)-tartrique pour former un précipité dans une solution, ledit précipité étant le tartrate de l'énantiomère de formule I ;
l'étape de séparation comprend la séparation du précipité de la solution ; et
le procédé comprenant en outre la transformation du tartrate du diastéréoisomère de formule I en la base libre correspondante.

10. Procédé suivant la revendication 1, dans lequel l'étape de réation comprend la réaction du mélange racémique avec l'acide L-(+)-tartrique pour former un précipité dans une solution, la solution comprenant à l'état dissous le tartrate de l'énantiomère de formule I ;
l'étape de séparation comprend la séparation du précipité de la solution et l'évaporation de la solution, laissant le sel de l'énantiomère de formule I ; et
le procédé comprenant en outre la transformation du tartrate de l'énantiomère de formule I en la base libre correspondante.

11. Sel d'un composé de formule 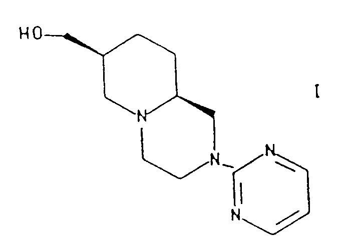 avec un acide choisi dans le groupe consistant en l'acide D-(-)-tartrique et l'acide L-(+)-tartrique.

12. Sel d'un composé de formule 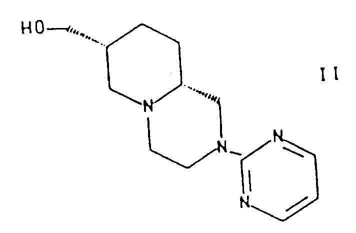 avec un acide choisi dans le groupe consistant en l'acide D-(-)-tartrique et l'acide L-(+)-tartrique.
